# EUROPEAN PATENT APPLICATION

(11) **EP 4 597 110 A2**
(43) Date of publication of application: **06.08.2025**
(21) Application number: 25154927.5
(22) Date of filing: 30.01.2025
(51) Int. Cl.: G01N 33/564, G01N 33/68

(54) **HYPERMOBILE EHLERS-DANLOS SYNDROME, HYPERMOBILITY SPECTRUM DISORDERS AND OSTEOARTHRITIS PLASMA MARKERS**

(30) Priority: 05.02.2024 IT 202400002376
(71) Applicant: Universita' degli studi di Brescia, 25121 Brescia (IT)
(72) Inventor: COLOMBI, Marina, I-25121 BRESCIA (IT); ZOPPI, Nicoletta, I-25121 BRESCIA (IT); RITELLI, Marco Giuseppe, I-25121 BRESCIA (IT); CHIARELLI, Nicola, I-25121 BRESCIA (IT)
(74) Representative: Comoglio, Elena

(57) **Abstract**

The present invention relates to *in vitro* methods for the diagnosis of hypermobile Ehlers-Danlos syndrome (hEDS), hypermobility spectrum disorders (HSD) and osteoarthritis (OA) based on the detection in plasma of characteristic patterns of fibronectin and collagen type I fragments as plasma markers.

## Description

### Technical field

The present invention falls within the field of diagnostics. It relates in particular to plasma markers for use in the diagnosis of hypermobile Ehlers-Danlos syndrome (hEDS), hypermobility spectrum disorders (HSD) and osteoarthritis (OA).

### Technical background

HEDS and HSD represent a major health problem globally, affecting millions of affected people. They are classified within the large group of inherited connective tissue diseases and are characterised mainly by joint hypermobility and musculoskeletal complications; their overall prevalence is estimated at 1 in 500 individuals. Unlike other forms of EDS, such as classical EDS (cEDS) and vascular EDS (vEDS), which are caused by specific genetic mutations involving collagen production and extracellular matrix (ECM) homeostasis, the molecular basis of hEDS and HSD remain unknown to date, despite a decade of intensive research. Consequently, the distinction between hEDS, HSD and related inherited connective tissue diseases to date is based solely on clinical criteria established in the 2017 International Classification of Ehlers-Danlos Syndromes [1]. According to these criteria, hEDS is defined by the simultaneous presence of generalised joint hypermobility according to Beighton's score, together with a combination of at least 5 out of 12 signs of multisystem involvement, positive family history and/or at least one musculoskeletal manifestation (pain for at least three months in the limbs, chronic generalised pain, recurrent joint dislocation or frank joint instability), excluding other inherited and acquired connective tissue diseases, including autoimmune rheumatological conditions such as RA and PsA. Patients who exhibit symptomatic joint hypermobility but do not meet the 2017 criteria for hEDS and do not show signs and symptoms of other inherited connective tissue diseases are classified as having HSD. A critical aspect is that these patients do not currently receive recognition by the National Health Service and are not eligible for genetic disease exemption and a specific care treatment plan.

Considering the wide clinical overlap between hEDS and HSD, as well as the lack of specific genetic tests and diagnostic biomarkers, there is much debate in the scientific community as to whether hEDS and HSD represent two different clinical entities or instead constitute a single phenotypic spectrum.

Previous studies carried out by the present inventors on dermal fibroblasts obtained from individuals suffering from hEDS and HSD have shown that, unlike cells from healthy donors, the cells from these patients share a marked disorganisation of several structural components of the extracellular matrix and characteristics typical of myofibroblasts. This pathological phenotype is induced in healthy donor cells by treatment with hEDS and HSD cell culture medium. Proteomic analysis of the hEDS and HSD cell culture medium revealed high levels of enzymes from the metalloprotease family that degrade the extracellular matrix and by the presence of fibronectin (UniProt ID: P02751), collagen type I (UniProt ID: P02452) and tenascin (UniProt ID: P24821) fragments [2,3]. Inhibition of metalloproteases is able to promote in hEDS and HSD cells the restoration of the organisation of an extracellular matrix similar to that of healthy donor cells and the attenuation of their myofibroblast characteristics [3].

Dermal fibroblasts derived from cEDS and vEDS patients exhibit disorganisation of extracellular matrix components due to the lack of collagen type V and III molecules, respectively, but do not show the distinctive features of myofibroblasts and the presence in the culture medium of the fragments identified in hEDS and HSD cells [2]. Furthermore, proteomic and transcriptomic studies confirmed that hEDS and HSD cells show common but distinct expression profiles compared to cells from healthy donors [4,5]. This concordance between hEDS and HSD observed in dermal fibroblasts cultured *in vitro* was also recently confirmed in a clinical study on a large cohort of hEDS and HSD patients. In particular, the inventors demonstrated that the multisystem manifestations of these patients represent a spectrum of overlapping presentations rather than two distinct conditions, highlighting the inadequacy of the 2017 diagnostic criteria for hEDS and the need for a reclassification of hEDS and HSD [6].

In summary, the clinical diagnostic criteria laid down in the 2017 International Classification of Ehlers-Danlos Syndromes have proved unsatisfactory, nor are there currently any known diagnostic markers or specific molecular tests for these diseases.

Other conditions characterised by joint deterioration associated with inflammation and joint pain are also known, such as RA, PsA and OA [7-9].

As with hEDS and HSD, the diagnosis of these degenerative joint conditions is primarily based on the clinical presentation of patients. In particular, for PsA and OA, in addition to clinical and radiographic evaluation, no specific diagnostic tests are available [8,9]. For RA, the presence of positivity to rheumatoid factor and anti-citrullinated protein antibodies may contribute to a more accurate diagnosis of the disease. However, these serological markers are not specific, as they can also be detected in other autoimmune diseases, following infections and/or due to other environmental and genetic factors [7].

Although the degradation of structural components of cartilage and bone in joints, such as collagen, fibronectin and tenascin, is known to occur in these inflammatory diseases, for which specific fragments with pro-inflammatory activity have been described that initiate a degradative feedback loop contributing to chronic synovitis and progressive cartilage and bone damage, there are no data on the presence of these protein fragments in plasma.

### Summary of the invention

There is therefore a need to identify specific diagnostic markers for hEDS and HSD, which will allow patients with these diseases to be distinguished from healthy individuals and from patients with other connective tissue and rheumatological diseases with common complications.

This and other needs are now met by the present invention, which makes available an *in vitro* method for the diagnosis of hypermobile Ehlers-Danlos syndrome (hEDS) or hypermobility spectrum disorders (HSD) as defined in claim 1.

As will be explained below, in the course of their studies to identify diagnostic markers for hEDS and HSD, these inventors also identified some specific markers for diseases with common joint complications, in particular osteoarthritis (OA), rheumatoid arthritis (RA) and psoriatic arthritis (PsA), which can therefore be used in the diagnosis of these diseases.

The scope of the present invention therefore also includes an *in vitro* method for the diagnosis of osteoarthritis (OA) as defined in the appended claim 3.

Further features and advantages of the present invention are defined in the dependent claims.

The appended claims form an integral part of the present disclosure.

An *in vitro* method for the diagnosis of rheumatoid arthritis (RA) or psoriatic arthritis (PsA) based on detecting in a plasma sample of a subject the presence of a tenascin fragment having a relative molecular mass of 38 kDa and/or a tenascin fragment having a relative molecular mass of 58 kDa, and optionally also the presence of a collagen type I fragment having a relative molecular mass of 45 kDa and/or a collagen type I fragment having a relative molecular mass of 60 kDa and/or a collagen type I fragment having a relative molecular mass of 80 kDa, is also disclosed but not claimed.

### Detailed description of the invention

The present invention is the result of studies carried out by the present inventors, who investigated the presence of connective tissue components degradation products, in particular fibronectin, type I collagen and tenascin, in the plasma of patients affected by hEDS and HSD and in the plasma of patients affected by other conditions characterised by joint deterioration associated with inflammation and joint pain, such as RA, PsA and OA

With their studies, the inventors identified the presence of various combinations of fibronectin, type I collagen or tenascin fragments in the plasma of subjects affected by hEDS, HSD, RA, PsA and OA, which are absent in the plasma of healthy donors. This approach makes a significant contribution to the definition of distinctive plasma biomarkers, which are of help in the differential diagnosis and open new perspectives for the personalised management and treatment of these complex and clinically overlapping conditions.

More specifically, the inventors detected the presence of characteristic patterns of fibronectin, type I collagen and tenascin fragments in the plasma of the patients analysed, which allow the differential diagnosis of hEDS and HSD, of RA and PsA and of OA.

The characteristic fragment patterns of each of the above diseases were identified by the present inventors by gel electrophoresis and were defined in terms of relative molecular mass.

In the case of a protein or a fragment of a protein obtained by gel electrophoresis, the relative molecular mass is a measure of the mass and can be determined by comparing the mobility of the protein or fragment under investigation with that of a series of standard proteins whose relative molecular mass is known, separated on the same gel. The relative molecular mass of a protein of interest, or one of its fragments, is then deduced by extrapolating its position on the calibration curve.

The characteristic fragment patterns identified by the present inventors in the plasma of the patiernts are described below.

The presence of a fibronectin fragment with a relative molecular mass of 52 kDa and a type I collagen fragment with a relative molecular mass of 45 kDa, observed in the plasma of all hEDS and HSD patients, demonstrates that these two diseases share a pathological process characterised by the degradation of structural components of the ECM of connective tissues. It is relevant to note that, contrary to previous *in vitro* studies, no tenascin fragments were found in the plasma of hEDS and HSD patients, which in contrast were present in the fibroblast culture medium.

Since the combination of a fibronectin fragment with a relative molecular mass of 52 kDa and a type I collagen fragment with a relative molecular mass of 45 kDa was constant in all samples from patients affected by hEDS and HSD, but was not observed in healthy individuals nor in patients affected by OA, PsA, RA, cEDS and vEDS, the combination of these fragments represents a plasma biomarker specific for hEDS and HSD.

The presence of the type I collagen fragment with a relative molecular mass of 45 kDa observed in the plasma of some patients with PsA and RA suggests that this fragment per se is not specific for hEDS and HSD. In contrast, the fibronectin fragment with a relative molecular mass of 52 kDa is per se specific for samples from patients with hEDS and HSD. Its presence in plasma, alone or in combination with the type I collagen fragment with a relative molecular mass of 45 kDa, may therefore be included as a diagnostic criterion in the ongoing classification of hEDS and HSD, helping to unify the two diseases on a phenotypic continuum.

With regard to OA, the presence of the fibronectin fragment with a relative molecular mass of 38 kDa emerged as a specific plasma marker of this disorder. The presence of the type I collagen fragment with a relative molecular mass of 30 kDa also emerged as a specific marker for this disorder. The presence of the fibronectin fragment with a relative molecular mass of 38 kDa, in combination with the type I collagen fragment with a relative molecular mass of 30 kDa, was also constant in all samples from patients with OA, but was not observed in healthy individuals or in patients with hEDS, HSD, PsA, RA, cEDS and vEDS, so that the combination of these fragments is also a specific plasma biomarker for OA. No tenascin fragments were observed.

With regard to the diagnosis of arthritis (which is outside the scope of the present invention), the presence of tenascin fragments in plasma emerged as a specific pattern of these rheumatological diseases. The tenascin fragment having a relative molecular mass of 58 kDa was more specific for PsA, as it was present in 95% of the samples, with only 2/40 samples showing the fragment having a relative molecular mass of 38 kDa. In contrast, the 38 kDa relative molecular mass fragment was more specific to RA (present in 77.5% of samples), with only 3/40 samples showing the 58 kDa relative molecular mass fragment and 6/40 showing both fragments. No fibronectin fragments were observed. With regard to type I collagen fragments, their absence seems to be indicative of PsA, as they were found in 87.5% (35/40) of the patients, with only 5/40 samples showing the 45 kDa relative molecular mass fragment. In contrast, several type I collagen fragments were detected in 90% of patients with RA, in various combinations, with only 4/40 samples showing no fragments.

The invention is further described in the following experimental example and related drawings, which are provided for illustrative purposes only and are not limiting the scope of the invention as defined in the appended claims.

### Drawings description

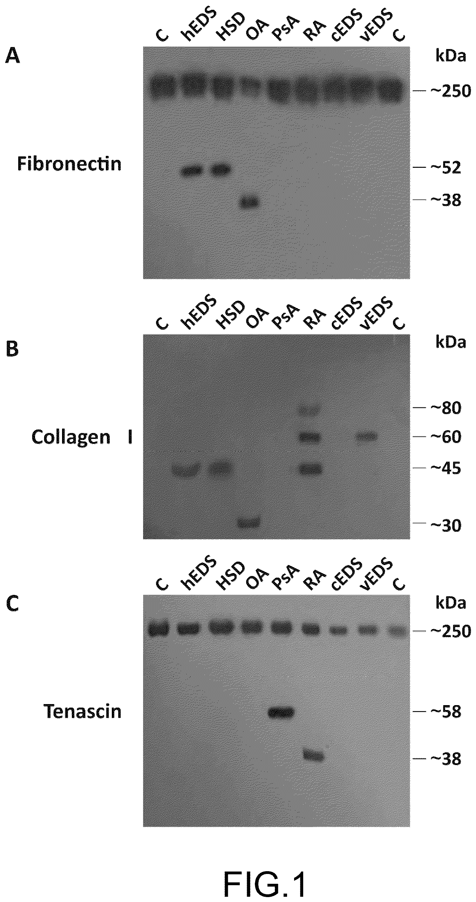

Figure 1. Representative images of the patterns of fibronectin (A), collagen type I (B) and tenascin (C) fragments most frequently detected by Western blotting with specific antibodies in the plasma of control individuals (C) and patients with hypermobile Ehlers-Danlos syndrome (hEDS), disorders in the joint hypermobility spectrum (HSD), osteoarthritis (OA), psoriatic arthritis (PsA), rheumatoid arthritis (RA), classic Ehlers-Danlos syndrome (cEDS) and vascular Ehlers-Danlos syndrome (vEDS).

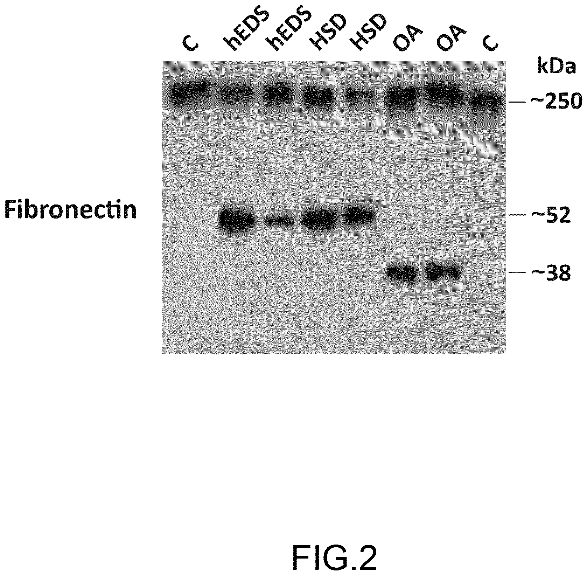

Figure 2. Representative image of the patterns of fibronectin fragments with a relative molecular mass of 52 kDa and 38 kDa identified in the plasma of patients with hypermobile Ehlers-Danlos syndrome (hEDS), hypermobility spectrum disorders articular (HSD) and patients with osteoarthritis (OA) by Western blotting with the monoclonal antibody f29 recognising the gelatin/collagen binding domain, respectively. C: plasma from control individuals.

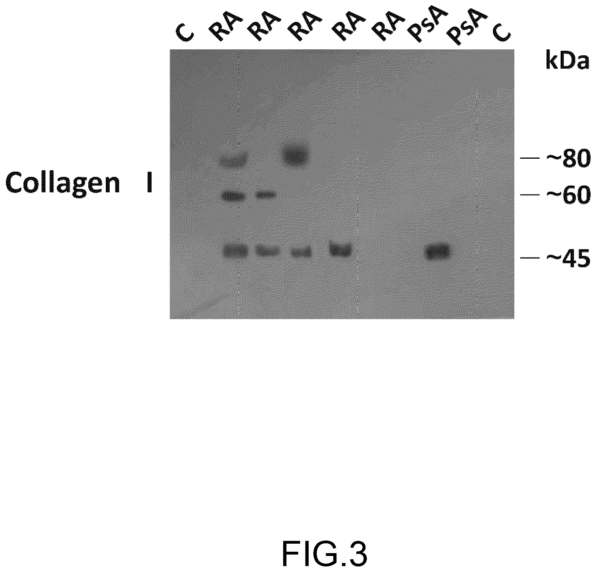

Figure 3. Representative image of the patterns of type I collagen fragments observed in the plasma of patients with rheumatoid arthritis (RA) and psoriatic arthritis (PsA). C: plasma from control individuals.

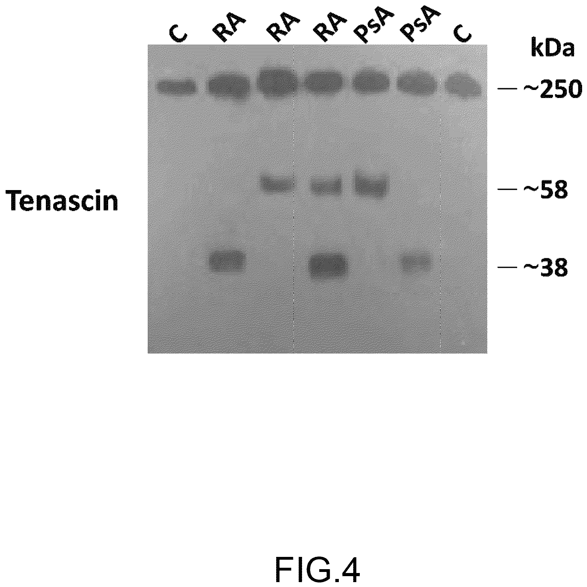

Figure 4. Representative image of the tenascin fragment patterns observed in the plasma of patients with rheumatoid arthritis (RA) and psoriatic arthritis (PsA). C: plasma from control individuals.

### Experimental example

### Methods

### Patient recruitment and blood sampling

Patients over 18 years of age with hEDS (55), HSD (55) and healthy donors (129) were recruited at the U.O.C. of Dermatology of the Spedali Civili di Brescia. Patients received a clinical diagnosis of hEDS according to the 2017 Ehlers-Danlos syndromes nosology criteria [1]. Patients who did not meet these criteria were classified as HSD.

Adult patients with cEDS (10) and vEDS (12) were recruited from the U.O.C. of Dermatology of Spedali Civili di Brescia. Patients were clinically diagnosed with cEDS and vEDS according to the 2017 EDS nosology criteria [1] and were all characterised for the presence of a pathogenic variant in the COL5A1 and COL3A1 genes, respectively.

Adult patients suffering from RA (40) and PsA (40) were recruited at the O.U. of Rheumatology and Clinical Immunology of the Spedali Civili di Brescia and clinically classified according to ACR/EULAR [10] and CASPAR [11] classification criteria, respectively.

Adult patients with advanced OA (40) were recruited from the O.U. of Orthopaedics and Traumatology of the Hospital of Manerbio, ASST del Garda, prior to hip or knee prosthetic surgery.

For all healthy individuals recruited, the presence of the clinical manifestations of the indicated diseases was excluded.

The ASST Spedali Civili di Brescia Ethics Committee approved the study protocol on patients with hEDS, HSD, cEDS, vEDS and healthy individuals (NP5582), on patients with RA and PsA (NP5832) and on patients with OA (NP5850). All participants were informed about the purpose, risks and benefits of the study and signed consent to participate in accordance with current privacy legislation.

The study also included a validation cohort of hEDS patients (39), HSD patients (25) and healthy individuals (21) recruited in collaboration with the EDS Society as part of the international HEDGE project, with approval from the Genetic Alliance Institutional Review Board, federal registration number IORG0003358, project number EDS002.

The present invention was therefore made in accordance with the provisions of Art. 170-bis, para. 3 of the Italian Industrial Property Code concerning obtaining informed consent.

### Blood Samples and Methods

Peripheral venous blood samples of the study participants were collected in the morning using 2.7 ml K3-EDTA S-Monovette^{®} tubes (Sarstedt) and in 3 ml BD VacutainerTM tubes (Vacumed) with sodium heparin. The tubes were kept at room temperature and processed within two hours of collection. Plasma was obtained by centrifugation at room temperature at 2,500 g for 15 minutes. The plasma supernatant was divided into 300 ml aliquots and stored at -80°C until use.

In addition, a blood sample was collected in 3.5 ml Vacumed tubes with gel separator for the preparation of serum, which was obtained by centrifugation at room temperature at 2,500 g for 15 minutes, divided into 300 µl aliquots and stored at -80°C until use.

### Quantification of plasma and serum proteins

Total protein concentration in plasma and serum was determined with the Bicinchoninic Acid Protein Determination Kit (Catalogue No. BCA1-1KT, Sigma Aldrich-Merck Life Science).

### Protein analysis by Western

Plasma proteins (30 micrograms) were denatured for 7 minutes at 99°C in loading buffer: 0.1 M Tris HCl pH 8.4% SDS, 20% glycerol, 0.005% bromophenol blue, 10% betamercaptoethanol) and subjected to polyacrylamide gel electrophoresis (PAGE) in the presence of sodium dodecyl sulphate (SDS), an anionic detergent that negatively charges proteins according to their relative mass and allows their separation from the negative to the positive pole in an electric field (SDS-PAGE). Specifically, the gel consisted of a 4% stacking gel and an 8% polyacrylamide running gel. For the separation in the stacking gel a voltage of 50 V was applied for one hour and 45 minutes and for the running gel a voltage of 150 V was applied for one hour and 15 minutes in the running buffer 25 mM Tris, 200 mM glycine, 0.1% SDS.

Molecular weight markers Novex^{®} Sharp Pre-stained Protein Standard (Catalogue No. LC5800, Thermo Fisher Scientific) were loaded into each gel.

Proteins were transferred onto nitrocellulose sheets (0.45 micrometer, Amersham Protran, GE Healthcare Life Science) in transfer buffer (0.2 M NaH₂PO₄, 0.2 M Na₂HPO₄ pH 6.5) for 3 hours at constant 36 V; the sheet was saturated overnight at 37°C in a 5% skimmed milk powder solution (Ristora Italia), 0.1 M Tris HCl pH 8 in 20 mM Tris HCl pH 7.4, 150 mM NaCl (TBS) with the addition of 0.1% Tween 20 (v/v) (TBS-T).

Proteins transferred onto nitrocellulose sheets were immunoreacted for 3 hours at room temperature with the following primary antibodies:
- polyclonal antibody (obtained in rabbit) directed against human fibronectin (Catalogue No. F3648, Sigma Aldrich-Merck Life Science); dilution 1:1,000 in 5% skimmed milk in TBS-T;
- monoclonal antibody f29 (non-commercial), which recognises the N-terminal gelatin/collagen binding domain of human fibronectin [12]; undiluted;
- polyclonal antibody directed against human type I collagen (obtained in goats) (Catalogue No. AB758, Merck-Millipore); dilution 1:500 in 5% skimmed milk in TBS-T;
- monoclonal antibody clone BC-24 directed against human tenascin (Catalogue No. T2551 Sigma Aldrich-Merck Life Science), which recognises an epitope located in the N-terminal EGF-like domain present in all its isoforms; 1 pg/ml in 5% skimmed milk in TBS-T.

After 3 washes in TBS-T for 10 min at room temperature, the blots were reacted for 3 h at room temperature with the following horseradish peroxidase enzyme-conjugated secondary antibodies diluted 1:1,000 in 5% skimmed milk in TBS-T:
- antibody directed against rabbit immunoglobulin (Ig) obtained in goats (Catalogue No. A8275, Sigma Aldrich-Merck Life Science);
- antibody directed against mouse Ig obtained in sheep (Catalogue No. A5906, Sigma Aldrich-Merck Life Science);
- antibody directed against goat Ig obtained in rabbits (Catalogue No. 401515, Sigma Aldrich-Merck Life Science).

After 3 washes in TBS-T for 10 minutes at room temperature, antigen-antibody binding was detected as a chemiluminescent signal using SuperSignal West Pico PLUS Chemiluminescent Substrate (Catalogue No. 34580, Thermo Fisher Scientific). Specifically, one minute of incubation with the substrate, 3 seconds of exposure, 15 seconds of development and 3 minutes of fixation were required to detect fibronectin. For type I collagen, the incubation was one minute and 20 seconds, exposure 12 seconds, development 24 seconds and fixation 3 minutes. For tenascin, the incubation was one minute, exposure 4 seconds, development 19 seconds and fixation 3 minutes.

The bands highlighted by ECL were sized for comparison with molecular weight markers.

### Results

Figure 1 shows the most frequently observed fragment patterns in Western blotting in plasmas of healthy and affected individuals with hEDS, HSD, OA, PsA, RA, cEDS and vEDS using polyclonal anti-fibronectin (A), polyclonal anti-collagen type I (B) and monoclonal anti-tenascin (C) antibodies.

Table 1 lists all fibronectin, collagen type I and tenascin fragment patterns observed in order of frequency in the plasma of healthy individuals and patients with hEDS, HSD, OA, PsA, RA, cEDS and vEDS.

The anti-fibronectin antibody recognises the intact protein of approximately 250 kDa in the plasma of all tested individuals, healthy and patients. In the plasma of all patients suffering from hEDS (94) and HSD (80) the antibody detected a fragment with a relative molecular mass of 52 kDa. In the plasma of all OA patients (40) the antibody detects a fragment with a relative molecular mass of 38 kDa. In the plasma of healthy donors (150) and patients with PsA (40), RA (40), cEDS (10) and vEDS (12) the antibody does not detect fibronectin fragments (Figure 1A, Table 1).

The intact fibronectin (250 kDa) and its fragments with relative molecular masses of 52 and 38 kDa, observed in patients with hEDS, HSD and OA, respectively, are also evidenced by the monoclonal antibody f29, which recognises the N-terminal gelatin/collagen binding domain (Figure 2).

The anti-collagen type I antibody showed no intact protein in any of the samples tested. In the samples of all patients with hEDS and HSD, a fragment with a relative molecular mass of 45 kDa is observed. In the plasma of all patients with OA, a fragment with a relative molecular mass of 30 kDa is present. In the majority of samples from patients with PsA (35/40, 87.5 %), no type I collagen fragments were observed and only in 5/40 samples (12.5 %) was the fragment with a relative molecular mass of 45 kDa observed as in the samples from hEDS and HSD patients. The fragment with a relative molecular mass of 45 kDa is also present in the majority of samples from RA patients (36/40, 90%); In particular, this fragment is present alone in 3/36 samples, in combination with a fragment having a relative molecular mass of 60 kDa (in 10/36 samples), in combination with a fragment having a relative molecular mass of 80 kDa (in 7/36 samples), or in combination with both fragments having a relative molecular mass of 60 and 80 kDa (in 16/36 samples). In 4/40 (10%) samples from patients with RA, no type I collagen fragment was detected (Figure 3). In the plasma of all patients with vEDS, the fragment with a relative molecular mass of 60 kDa is observed. In the plasma of all healthy individuals and cEDS patients, no type I collagen fragment is evident (Figure 1B, Table 1).

The anti-tenascin antibody recognises the intact protein of approximately 250 kDa in the plasma of all tested individuals, healthy and patients. In the samples of all patients with PsA and RA, the presence of tenascin fragments is observed. In particular, in the majority of patients with PsA (38/40, 95%) the presence of a fragment with a relative molecular mass of 58 kDa and in 2/40 (0.5%) the presence of a fragment with a relative molecular mass of 38 kDa was observed. The fragment with a relative molecular mass of 38 kDa is present in the majority of patients with RA (37/40, 92.5%); in particular, this fragment is present alone in 31/37 samples or in combination with the fragment with a relative molecular mass of 58 kDa (in 6/37 samples). In 3/40 samples (7.5 %), only one fragment with a relative molecular mass of 58 kDa is present (Figure 4). In the plasma of all healthy individuals and the hEDS, HSD, OA, cEDS and vEDS patients, no tenascin fragment was detected (Figure 1C, Table 1).

Table 2 shows the different combinations of fibronectin, type I collagen and tenascin fragments in order of frequency identified in the plasma of healthy individuals and patients with hEDS, HSD, OA, PsA, RA, cEDS and vEDS.

Samples from patients with hEDS and HSD all show the combination of the fibronectin fragment with a relative molecular mass of 52 kDa and the type I collagen fragment with a relative molecular mass of 45 kDa, with the absence of tenascin fragments.

Samples from OA patients all show the combination of the fibronectin fragment having a relative molecular mass of 38 kDa and the type I collagen fragment having a relative molecular mass of 30 kDa, with the absence of tenascin fragments.

The most frequent combination in samples from patients with PsA is the absence of fibronectin and type I collagen fragments and the presence of the tenascin fragment with a relative molecular mass of 58 kDa (33/40, 82.5%). The combination of the absence of fibronectin fragments and the presence of the type I collagen fragment with a relative molecular mass of 45 kDa and the tenascin fragment with a relative molecular mass of 58 kDa was observed in 5/40 (12.5%) patients. The combination of the absence of fibronectin and type I collagen fragments and the presence of the tenascin fragment with a relative molecular mass of 38 kDa was observed in 2/40 (5%) patients.

The most frequent combination in samples from patients with RA is the absence of fibronectin fragments, the presence of type I collagen fragments with a relative molecular mass of 45, 60 and 80 kDa and the presence of the tenascin fragment with a relative molecular mass of 38 kDa (12/40, 30%). The combination of the absence of fibronectin fragments, the presence of collagen type I fragments having a relative molecular mass of 45 and 60 kDa and the presence of the tenascin fragment having a relative molecular mass of 38 kDa is found in 9/40 (22.5%) patients. The combination of the absence of fibronectin fragments, the presence of collagen type I fragments with a relative molecular mass of 45 and 80 kDa and the tenascin fragment with a relative molecular mass of 38 kDa is observed in 6/40 (15%) patients. The combination of the absence of fibronectin fragments and the presence of collagen type I fragments with a relative molecular mass of 45, 60 and 80 kDa and tenascin fragments with a relative molecular mass of 38 and 58 kDa is observed in 4/40 (10%) patients. The combination of the absence of fibronectin and type I collagen fragments and the presence of the tenascin fragment having a relative molecular mass of 58 kDa is observed in 3/40 (7.5%) patients. The same number of patients shows the combination of the absence of fibronectin fragments and the presence of the type I collagen fragment with a relative molecular mass of 45 kDa and the tenascin fragment with a relative molecular mass of 38 kDa. In only one patient (2.5%) was one of the following three combinations observed: (i) absence of fibronectin fragments, presence of collagen type I fragments having relative molecular masses of 45 and 60 kDa and tenascin fragments having relative molecular masses of 38 and 58 kDa; (ii) absence of fibronectin fragments, presence of collagen type I fragments having a relative molecular mass of 45 and 80 kDa and tenascin fragments having a relative molecular mass of 38 and 58 kDa; (iii) absence of fibronectin and collagen type I fragments and presence of the tenascin fragment having a relative molecular mass of 38 kDa.

No fibronectin, type I collagen and tenascin fragments were detected in all samples from patients with cEDS.

In all samples from patients with vEDS, only the presence of the type I collagen fragment with a relative molecular mass of 60 kDa was evident.

No fibronectin, collagen type I and tenascin fragments were detected in plasma collected in heparin tubes and in serum.

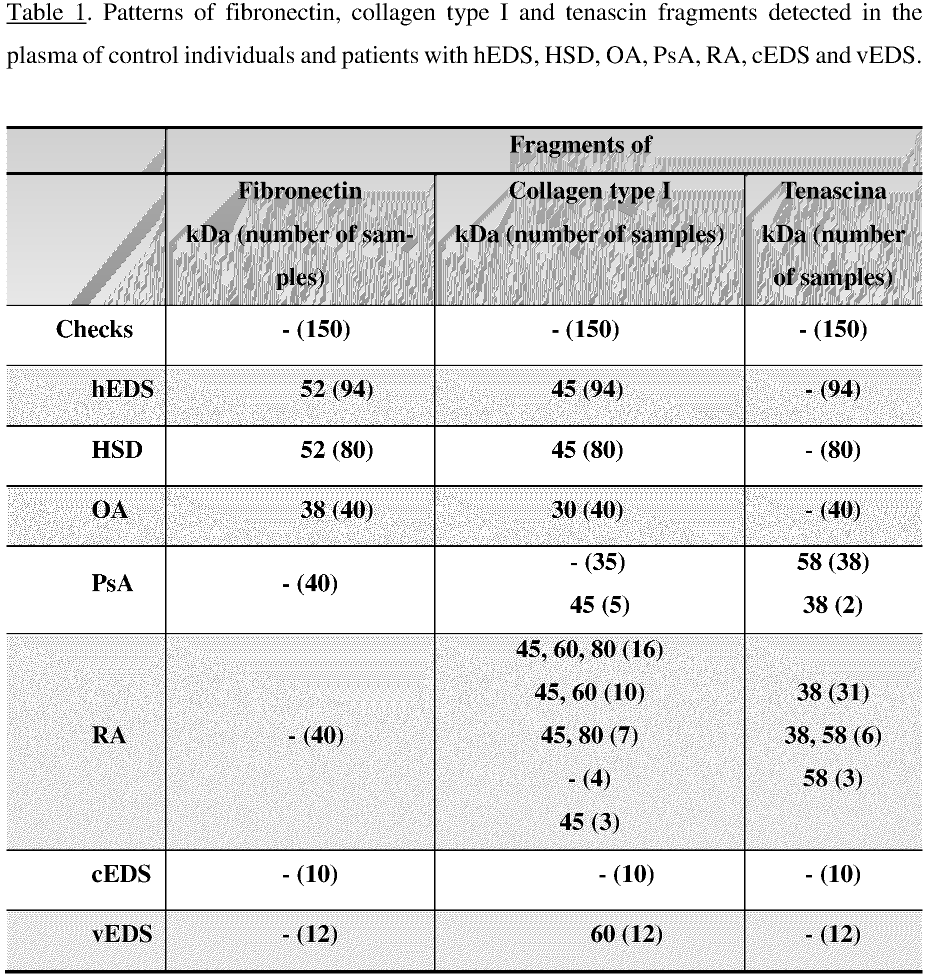

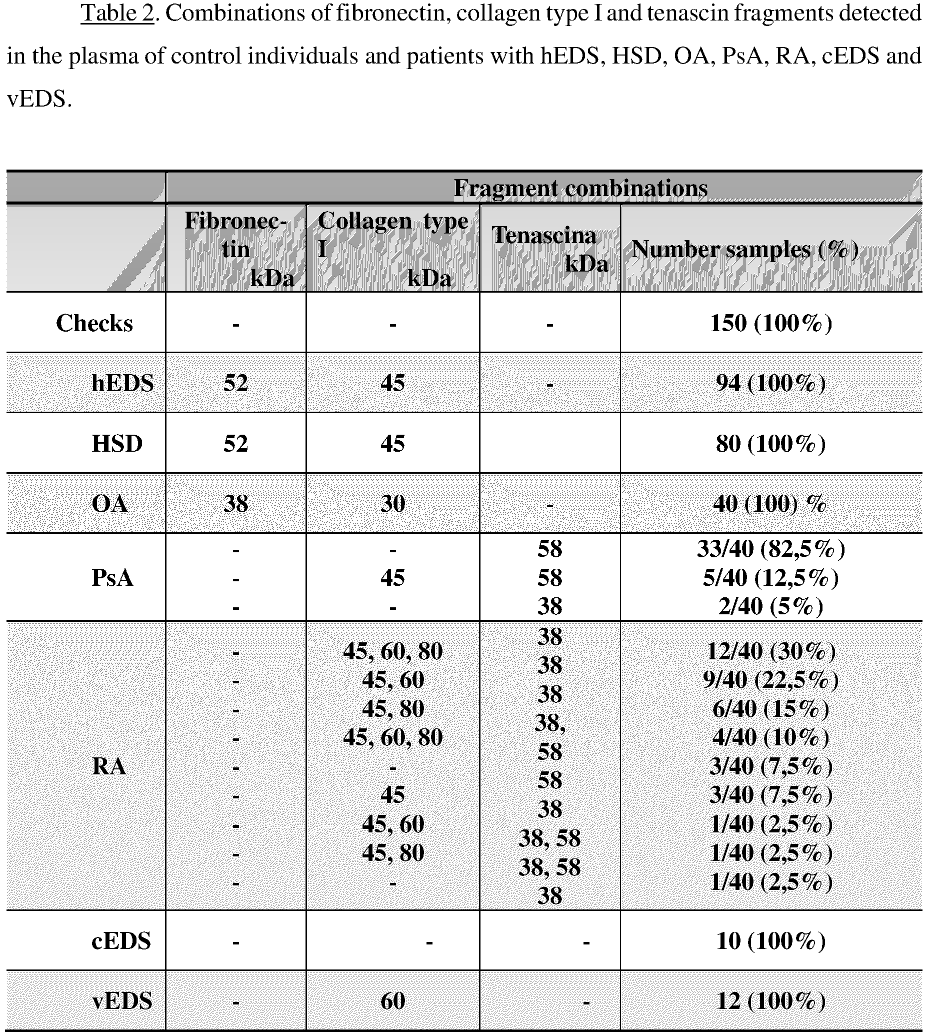

### References

1. Malfait F, Francomano C, Byers P, Belmont J, Berglund B, Black J, Bloom L, Bowen JM, Brady AF, Burrows NP, Castori M, Cohen H, Colombi M, Demirdas S, De Backer J, De Paepe A, Fournel-Gigleux S, Frank M, Ghali N, Giunta C, Grahame R, Hakim A, Jeu-nemaitre X, Johnson D, Juul-Kristensen B, Kapferer-Seebacher I, Kazkaz H, Kosho T, La-vallee ME, Levy H, Mendoza-London R, Pepin M, Pope FM, Reinstein E, Robert L, Rohrbach M, Sanders L, Sobey GJ, Van Damme T, Vandersteen A, van Mourik C, Voer-mans N, Wheeldon N, Zschocke J, Tinkle B. The 2017 international classification of the Ehlers-Danlos syndromes. Am. J. Med. Genet. Part C Semin. Med. Genet. 2017, 175: 8-26.
2. Zoppi N, Chiarelli N, Binetti S, Ritelli M, Colombi M. Dermal fibroblast-to-myofibroblast transition sustained by αvβ3 integrin-ILK-Snail1/Slug signaling is a common feature for hypermobile Ehlers-Danlos syndrome and hypermobility spectrum disorders. Biochim Biophys Acta Mol Basis Dis. 2018,1864: 1010-1023.
3. Chiarelli N, Zoppi N, Venturini M, Capitanio D, Gelfi C, Ritelli M, Colombi M. Matrix metalloproteinases inhibition by doxycycline rescues extracellular matrix organization and partly reverses myofibroblast differentiation in hypermobile Ehlers-Danlos syndrome dermal fibroblasts: a potential therapeutic target for patients' management? Cells 2021 ,10(11): 3236.
4. Chiarelli N, Zoppi N, Ritelli M, Venturini M, Capitanio D, Gelfi C, Colombi M. Biological insights into the pathogenesis of hypermobile Ehlers-Danlos syndrome from prote-ome profiling of patients' dermal myofibroblasts. Biochim Biophys Acta Mol Basis Dis. 2021, 1867(4): 166051.
5. Ritelli M, Chiarelli N, Cinquina V, Zoppi N, Bertini V, Venturini M, Colombi M. RNA-Seq of Dermal fibroblasts from patients with hypermobile Ehlers-Danlos Syndrome and Hypermobility Spectrum Disorders supports their categorization as a single entity with involvement of extracellular matrix degrading and proinflammatory pathomechanisms. Cells 2022, 11(24): 4040.
6. Ritelli M, Chiarelli N, Cinquina V, Vezzoli M, Venturini M, Colombi M. Looking back and beyond the 2017 diagnostic criteria for hypermobile Ehlers-Danlos syndrome: a retrospective cross-sectional study from an Italian reference centre. Am J Med Genet 2023, 1870(1): 166915.
7. Smolen JS, Aletaha D, Barton A, Burmester GR, Emery P, Firestein GS, Kavanaugh A, McInnes IB, Solomon DH, Strand V, Yamamoto K. Rheumatoid arthritis. Nat Rev Dis Primers. 2018, 4:18001.
8. FitzGerald O, Ogdie A, Chandran V, Coates LC, Kavanaugh A, Tillett W, Leung YY, deWit M, Scher JU, Mease PJ. Psoriatic arthritis. Nat Rev Dis Primers. 2021, 7: 59.
9. Martel-Pelletier J, Barr AJ, Cicuttini FM, Conaghan PG, Cooper C, Goldring MB, Goldring SR, Jones G, Teichtahl AJ, Pelletier JP. Osteoarthritis. Nat. Rev. Dis. Prim. 2016, 2: 16072.
10. Aletaha D, Neogi T, Silman AJ, Funovits J, Felson DT, Bingham CO 3rd, Birnbaum NS, Burmester GR, Bykerk VP, Cohen MD, Combe B, Costenbader KH, Dougados M, Emery P, Ferraccioli G, Hazes JM, Hobbs K, Huizinga TW, Kavanaugh A, Kay J, Kvien TK, Laing T, Mease P, M6nard HA, Moreland LW, Naden RL, Pincus T, Smolen JS, Stani-slawska-Biernat E, Symmons D, Tak PP, Upchurch KS, Vencovsky J, Wolfe F, Hawker G. 2010 Rheumatoid arthritis classification criteria: an American College of Rheumatology/European League Against Rheumatism collaborative initiative. Ann Rheum Dis. 2010,69(9): 1580-8.
11. Taylor W, Gladman D, Helliwell P, Marchesoni A, Mease P, Mielants H; CASPAR Study Group. Classification criteria for psoriatic arthritis: development of new criteria from a large international study. Arthritis Rheum. 2006,54(8): 2665-73.
12. Colombi M, Zoppi N, De Petro G, Marchina E, Gardella R, Tavian D, Ferraboli S, Barlati S. Matrix assembly induction and cell migration and invasion inhibition by a 13-amino acid fibronectin peptide. J Biol Chem. 2003, 278(16): 14346-55.

## Claims

1. An *in vitro* method for the diagnosis of hypermobile Ehlers-Danlos syndrome or hypermobility spectrum disorders, comprising detecting in a plasma sample of a subject the presence of a fibronectin fragment having a relative molecular mass of 52 kDa, the presence of said fibronectin fragment having a relative molecular mass of 52 kDa being indicative of hypermobile Ehlers-Danlos syndrome or hypermobility spectrum disorders.

2. The *in vitro* method according to claim 1, further comprising detecting in the plasma sample of the subject the presence of a type I collagen fragment having a relative molecular mass of 45 kDa, the presence of said type I collagen fragment having a relative molecular mass of 45 kDa in combination with the presence of said fibronectin fragment having a relative molecular mass of 52 kDa being indicative of hypermobile Ehlers-Danlos syndrome or hypermobility spectrum disorders.

3. An *in vitro* method for the diagnosis of osteoarthritis, comprising detecting in a plasma sample of a subject the presence of a fibronectin fragment having a relative molecular mass of 38 kDa and/or a collagen type I fragment having a relative molecular mass of 30 kDa, the presence of said fibronectin fragment having a relative molecular mass of 38 kDa and/or said collagen type I fragment having a relative molecular mass of 30 kDa being indicative of osteoarthritis.

4. The *in vitro* method according to any one of claims 1 to 3, wherein said fragments are detected by Western blotting.
